(19) Europäisches Patentamt
European Patent Office
Office européen des brevets

(11) EP 0 960 874 B1

(12) EUROPÄISCHE PATENTSCHRIFT

(45) Veröffentlichungstag und Bekanntmachung des Hinweises auf die Patenterteilung:
**07.03.2001 Patentblatt 2001/10**

(51) Int Cl.⁷: **C07C 51/215**, C07C 51/25, C07C 53/08, B01J 23/22, B01J 37/02, B01J 23/16, B01J 23/847, B01J 23/887, B01J 23/68

(21) Anmeldenummer: **99109226.3**

(22) Anmeldetag: **21.05.1999**

(54) **Verfahren zur Herstellung von Essigsäure durch Gasphasenoxidation von gesättigten C4-Kohlenwasserstoffen und deren Gemische mit ungesättigten C4-Kohlenwasserstoffen**

Method of production of acetic acid by gas phase oxidation of saturated C4-hydrocarbons and their mixtures with unsaturated C4-hydrocarbons

Procédé de préparation d' acide acétique par oxidation en phase gaseuze des hydrocarbures en C4 saturés et ses melanges avec des hydrocarbures en C4 insaturés

(84) Benannte Vertragsstaaten:
**AT BE CH DE ES FI FR GB IT LI NL SE**

(30) Priorität: **22.05.1998 DE 19823052**

(43) Veröffentlichungstag der Anmeldung:
**01.12.1999 Patentblatt 1999/48**

(73) Patentinhaber: **Consortium für elektrochemische Industrie GmbH**
**81379 München (DE)**

(72) Erfinder:
• **Rüdinger, Christoph, Dr.**
**81497 München (DE)**
• **Eberle, Hans-Jürgen, Dr.**
**81477 München (DE)**

(74) Vertreter: **Schuderer, Michael, Dr. et al**
**Wacker-Chemie GmbH**
**Zentralabteilung Patente**
**Marken und Lizenzen**
**Hanns-Seidel-Platz 4**
**81737 München (DE)**

(56) Entgegenhaltungen:
**DE-A- 2 041 073**      **DE-A- 19 649 426**
**US-A- 3 464 930**      **US-A- 4 066 704**
**US-A- 4 257 921**

• **MARK, OTHMER, OVERBERGER, SEABORG: "Kirk-Othmer Encyclopedia of Chemical Technology, Ed. 3, Vol. 1" 1978 , WILEY & SONS , NEW YORK, US XP002112493 * Seite 136, Absatz 3 - Seite 138, Absatz 1 ***

**Beschreibung**

Die Erfindung betrifft Verfahren zur Herstellung von Essigsäure durch Gasphasenoxidation von gesättigten $C_4$-Kohlenwasserstoffen und deren Gemische mit ungesättigten $C_4$-Kohlenwasserstoffen unter Verwendung eines Schalenkatalysators.

Es ist bekannt, daß Essigsäure durch Gasphasenoxidation von $C_2$-, $C_3$- und $C_4$-Körpern mittels eines Katalysators hergestellt werden kann. Bisher konnte jedoch noch kein wirtschaftlich und betriebstechnisch voll befriedigendes Verfahren gefunden werden.

Die DE-B 1279011 beschreibt ein Verfahren zur Herstellung von Essigsäure durch katalytische Gasphasenoxidation von Buten mit Sauerstoff unter Verwendung von Aluminium- und Titanvanadatkatalysatoren. Diese Katalysatoren werden durch Ausfällen der Mischoxide aus den entsprechenden Lösungen hergestellt, wobei die Mischoxide gegebenenfalls noch mit inerten Materialien wie Kieselsäure vermischt werden können. Der Katalysator wird als feinteiliges Pulver in Wirbelbettreaktoren eingesetzt. Nachteilig bei derartigen Vollkontakt-Katalysatoren ist der hohe Grad an Totaloxidation.

Zur Verbesserung der Ausbeute derartiger Katalysatoren wird in der DE-A 2016681 vorgeschlagen, die Katalysatoren vor der Calcinierung mit einem Oxidationsmittel vorzubehandeln.

DE-A 2041073 beschreibt ein Verfahren zur Herstellung von Essigsäure durch Gasphasenoxidation von Buten unter Verwendung eines Schalenkatalysators, bestehend aus einer inerten Träger und einer darauf aufgebrachten katalytischen Masse aus Titandioxid und Vanadiumpentoxid. Als geeignete Ausgangsstoffe werden Buten und "C-Schnitte" genannt, die als Verunreinigungen auch gesättigte Kohlenwasserstoffe enthalten können.

In der DE-A 2354425 (US-A 3954857) wird zur Verbesserung der Selektivität die Behandlung des calcinierten Titan-Vanadium-Mischkatalysators mit Salzsäure vorgeschlagen. Die Katalysatoren werden als Vollkontakt, gegebenenfalls im Gemisch mit inerten Trägermaterialien wie Kieselsäure, eingesetzt.

Ein weiterer aus dem Stand der Technik bekannter Ansatzpunkt, zur Verbesserung der Aktivität von Titan-Vanadium-Mischkatalysatoren bei der Gasphasenoxidation von Butenen zu Essigsäure, ist der Einsatz von $TiO_2$ in definierter Kristallform oder mit definierter Oberfläche.

In der DE-A 2026744 (US-A 3917682) werden Ti-V-Mischkatalysatoren beschrieben, deren $TiO_2$-Komponente überwiegend als Rutil vorliegt. Die Katalysatoren können in Pulverform oder zu Formkörpern gepreßt eingesetzt werden.

Aus der US-A 4448897 sind Ti-V-Katalysatoren zur Butenoxidation bekannt, welche $TiO_2$ mit einer BET-Oberfläche größer 40 $m^2$/g enthalten. Die Katalysatoren werden ebenfalls in Pulverform oder als Preßling eingesetzt.

Aus dem Stand der Technik ist weiter bekannt, die Selektivität von Ti-V-Katalysatoren bei der Butenoxidation dadurch zu verbessern, daß man den Titandioxid-Anteil ganz oder teilweise durch andere Metalloxide substituiert.

Die DE-A 2110876 (GB-A 1333306) beispielsweise beschreibt Katalysatoren, welche Oxide von Molybdän, Zinn und Vanadium als Aktivkomponenten enthalten. Die Katalysatoren werden in Pulverform eingesetzt, wobei der Mischoxid-Katalysator gegebenenfalls auch auf feinteilige Trägermaterialien wie Siliciumdioxid aufgetragen werden kann.

Aus der US-A 4146734 ist bekannt, Vanadium-Mischoxide einzusetzen, welche mit Cer und weiteren Übergangsmetalloxiden dotiert sind. Der Katalysator wird als feinteilges Granulat eingesetzt, kann aber auch als Präzipitat auf feinteilige, inerte Träger aufgetragen werden.

Aus der DE-A 2235103 sind Ti-V-Mischoxid-Katalysatoren zur Gasphasenoxidation von Butenen in Form von Trägerkatalysatoren bekannt, bei denen ein vorgeformter poröser Träger mit der Mischlösung der Katalysatorkomponenten getränkt wird.

Allen diesen Verfahren ist gemeinsam, daß es sich um Vollkontakt-Katalysatoren handelt, bei denen die Aktivkomponenten selbst als Pulver oder Preßlinge eingesetzt werden, oder mit feinteiligen Trägermaterialien verdünnt als Pulver oder Preßling eingesetzt werden. Als Vollkontakte sind auch mit Aktivkomponente durchtränkte poröse Träger gemäß DE-A 2235103 zu verstehen, da auch hier das gesamte Katalysatorvolumen katalytisch aktiv ist. Nachteilig bei Vollkontakt-Katalysatoren ist der hohe Grad an Totaloxidation und die bei hohen Umsätzen schlechte Beherrschbarkeit der Oxidationsreaktion.

Es bestand daher die Aufgabe, ein Verfahren und einen Katalysator zur Herstellung von Essigsäure durch Gasphasenoxidation von gesättigten $C_4$-Kohlenwasserstoffen und deren Gemisch mit ungesättigten $C_4$-Kohlenwasserstoffen zur Verfügung zu stellen, welche zu besserer Ausbeute und besserem Betriebsverhalten bei der Oxidationsreaktion führen.

Gegenstand der Erfindung ist ein Verfahren zur Herstellung von Essigsäure durch Gasphasenoxidation von gesättigten $C_4$-Kohlenwasserstoffen und deren Gemische mit ungesättigten $C_4$-Kohlenwasserstoffen in einem Rohrreaktor unter Verwendung eines Schalenkatalysators, bestehend aus einem inerten unporösen Trägerkörper und einer auf die äußere Oberfläche des Trägerkörpers aufgebrachten katalytisch aktiven Mischoxidmasse, welche

a) ein oder mehrere Oxide aus der Gruppe Titandioxid, Zirkoniumdioxid, Zinndioxid, Aluminiumoxid und

b) 0,1 bis 1,5 Gew.-%, bezogen auf das Gewicht der Komponente a) und pro $m^2$/g spezifischer Oberfläche der Komponente a), Vanadiumpentoxid enthält, wobei ein Gasgemisch, enthaltend Sauerstoff oder sauerstoffhaltiges Gas, ein oder mehrere $C_4$-Kohlenwasserstoffe und Wasserdampf, mit einem Volumenmischungsverhältnis $C_4$-Kohlenwasserstoff/Luft (Sauerstoff) von 0.2/99.8 bis 25/75 und einem Volumenmischungsverhältnis $C_4$-Kohlenwasserstoff/Wasserdampf von 1/1 bis 1/60, bei einer Temperatur von 100°C bis 400°C, und einem Überdruck von 0,2 bis 50 bar an dem Schalenkatalysator umgesetzt wird.

Bei dem erfindungsgemäßen Verfahren wird ein Gasgemisch, enthaltend Sauerstoff oder ein sauerstoffhaltiges Gas, bevorzugt Luft, ein oder mehrere $C_4$-Kohlenwasserstoffe, bevorzugt Butan und dessen Mischungen mit Buten, Wasserdampf und gegebenenfalls ein Inertgas, bei erhöhter Temperatur an dem Schalenkatalysator umgesetzt.

Die Gasphasenoxidation wird in gekühlten Rohrreaktoren, welche mit dem Schalenkatalysator befüllt sind und vom Reaktionsgemisch durchströmt werden, durchgeführt. Übliche Festbettreaktoren sind senkrecht angeordnete Rohrbündelreaktoren mit Rohrlängen von 1 m bis 10 m, einem Rohrinnendurchmesser von 10 bis 35 mm und einer Wandstärke von 1 bis 4 mm.

Als Wärmetauschermedien zur Kühlung sind besonders Wasser, Wärmeträgeröle und eutektische Salzschmelzen, beispielsweise Mischungen aus $KNO_3$/$NaNO_2$ geeignet.

Die Reaktionsrohre können gegebenenfalls mit Schalenkatalysatoren unterschiedlicher Gestalt und Dimension sowie unterschiedlicher Zusammensetzung der Aktivkomponenten bzw. der Schalen befüllt werden. Dabei können die Schalenkatalysatoren als statistische Mischung oder zonenweise in die Reaktionsrohre eingebracht werden.

Geeignete Edukte sind gesättigte Kohlenwasserstoffe und deren Gemische mit ungesättigten Kohlenwasserstoffen mit vier Kohlenstoffatomen oder Gasgemische die Kohlenwasserstoffe mit vier Kohlenstoffatomen enthalten. Unverzweigte $C_4$-Kohlenwasserstoffe liefern höhere Ausbeuten als verzweigte $C_4$-Kohlenwasserstoffe und Butadiene. Besonders bevorzugt sind n-Butan, 1-Buten, 2-Butene und deren Gemische.

Ein Vorteil der erfindungsgemäßen Verfahrensweise der Gasphasenoxidation von $C_4$-Kohlenwasserstoffen unter Verwendung des Schalenkatalysators besteht darin, daß auch Gasgemische eingesetzt werden können, welche Verbindungen enthalten, die nicht oder nur in geringem Maße oder in schlechten Ausbeuten zu Essigsäure reagieren. Es können somit auch billige Rohstoffgemische aus Raffinerien wie "$C_4$-Schnitt" (überwiegender Anteil an Butadien und i-Buten), "Raffinat 1" (überwiegender Anteil an i-Buten), "Raffinat 2" (überwiegender Anteil an 1-Buten und 2-Butenen) sowie Gemische, die neben $C_4$-Kohlenwasserstoffen lineare und/oder verzweigte und/oder cyklische Kohlenwasserstoffe mit mehr oder weniger als vier Kohlenstoffatomen wie z.B. Methan, Ethan, Ethen, Propen, Propan, Pentane, Pentene, Pentadiene, Cyclopentan, Cyclopenten, Cyclopentadien, Methylcyclopentan etc. enthalten als Ausgangsmaterial eingesetzt werden. Ebenso können Alkohole, Aldehyde, Ether, Ketone und Ester mit 1-8 Kohlenstoffatomen anwesend sein. Die genannten Rohstoffgemische können gegebenenfalls vor deren Einsatz auch einem Hydrier- oder Reinigungsschritt unterzogen werden.

Die Reaktionstemperatur für die Oxidation der Butan und/oder Buten/Sauerstoff(Luft)/Wasserdampf-Reaktionsgemische beträgt im allgemeinen 100°C bis 400°C, vorzugsweise 150°C bis 300°C.

Die Reaktion kann bei dem Staudruck, der sich durch die Durchströmung des Katalysatorbettes ergibt oder unter erhöhtem Druck durchgeführt werden. Bevorzugt wird bei 0,2 bis 50 bar, besonders bevorzugt bei 1 bis 16 bar Überdruck gearbeitet.

Das Volumenmischungsverhältnis C4-Kohlenwasserstoff/Luft (Sauerstoff) beträgt im allgemeinen 0,2/99,8 bis 25/75, bevorzugt 1/99 bis 5/95. Das Volumenmischungsverhältnis $C_4$-Kohlenwasserstoff/Wasserdampf beträgt im allgemeinen 1/1 bis 1/60, bevorzugt 1/5 bis 1/25. Die Raumgeschwindigkeit des Gasgemisches im Reaktor liegt bei 400 bis 10000 $h^{-1}$, vorzugsweise 600 bis 6000 $h^{-1}$ (Normbedingungen).

Nach der Umsetzung wird die gebildete Essigsäure durch Abkühlen und Niederschlagen oder durch Absorption in einem geeigneten Lösungsmittel abgetrennt. Die abgetrennte Essigsäure wird mit geeigneten Verfahren, beispielsweise Destillation oder Extraktion weiter gereinigt. Das Abgas kann im Kreislauf zurückgeführt werden.

Als einen weiteren Bestandteil der Erfindung wurde gefunden, daß für die Herstellung von Essigsäure durch Gasphasenoxidation von gesättigten Kohlenwasserstoffen mit vier Kohlenstoffatomen und deren Gemisch mit ungesättigten Kohlenwasserstoffen mit vier Kohlenstoffatomen besonders Schalenkatalysatoren geeignet sind, bei denen die aktive Masse als dünne Schicht auf einem unporösen Trägerkörper aufgebracht ist.

Die erfindungsgemäßen Schalenkatalysatoren bestehen aus einem inerten unporösen Trägerkörper und einer auf die äußere Oberfläche des Trägerkörpers aufgebrachten katalytisch aktiven Mischoxidmasse, welche

a) ein oder mehrere Oxide aus der Gruppe Titandioxid, Zirkoniumdioxid, Zinndioxid, Aluminiumoxid und

b) 0.1 bis 1.5 Gew.-%, bezogen auf das Gewicht der Komponente a)

und pro $m^2$/g spezifischer Oberfläche der Komponente a), Vanadiumpentoxid enthält.

Die Angabe Gew.-% bezogen auf das Gewicht der Komponente a) und pro $m^2/g$ spezifischer Oberfläche der Komponente a) bedeutet dabei, daß der einzusetzende Gewichtsanteil der Komponente b) von der spezifischen Oberfläche der Komponente a) abhängig ist. So ist beispielsweise bei einer spezifischen Oberfläche der Komponente a) von 10 $m^2/g$ der Anteil der Komponente b) 1 bis 15 Gew.-%, bezogen auf das Gewicht der Komponente a).

Geeignet ist $TiO_2$ sowohl in Rutil- als auch in Anatas-Form und deren Gemische. Bevorzugt wird als Komponente a) Titandioxid mit einer BET-Oberfläche von 20 bis 400 $m^2/g$, vorzugsweise 40 bis 300 $m^2/g$. Werden Gemische von Titandioxid mit Aluminiumoxid, Zirkoniumdioxid oder Zinndioxid eingesetzt, kann der Titandioxidanteil zu 5 bis 95 Gew.-%, vorzugsweise 5 bis 50 Gew.-%, durch Zirkoniumdioxid, Aluminiumoxid oder Zinndioxid ersetzt werden.

Als zusätzliche Komponente a) können noch ein oder mehrere Oxide aus der Gruppe Bor, Silicium, Hafnium, Niob, Wolfram, Lanthan und Cer enthalten sein. Bei der Dotierung der Komponente a) mit den genannten Oxiden sind diese im allgemeinen in einer Menge von 1 bis 30 Gew.-%, bezogen auf das Gesamtgewicht der Komponente a), enthalten.

Der Anteil der Komponente b) beträgt vorzugsweise 0,1 bis 1 Gew.-%, besonders bevorzugt 0,1 bis 0,5 Gew.-%, jeweils bezogen auf das Gewicht der Komponente a) und pro $m^2/g$ spezifischer Oberfläche der Komponente a).

Bei der Komponente b) kann gegebenenfalls ein Teil des Vanadiumpentoxids, vorzugsweise 10 bis 90 Gew.-%, durch ein oder mehrere Oxide von Molybdän, Chrom und Antimon ersetzt werden. Gegebenenfalls können als zusätzliche Komponente b) noch ein oder mehrere Oxide von Alkalimetallen, Elementen der 5. und 6. Hauptgruppe des Periodensystems der Elemente (PSE) und der Übergangsmetalle enthalten sein. Beispiele hierfür sind die Oxide von Lithium, Natrium, Kalium, Rubidium, Caesium, Phosphor, Wismut, Schwefel, Selen, Tellur, Mangan, Eisen, Cobalt, Palladium, Kupfer, Silber, Gold, Zink und Cadmium. Im allgemeinen beträgt die Menge dieser Dotierstoffe 0,005 bis 15 Gew.-%, berechnet als Oxide und bezogen auf das Gesamtgewicht der Komponente b). Der Anteil an Alkalimetalloxiden und Edelmetalloxiden beträgt dabei vorzugsweise 0,005 bis 1,0 Gew.-%.

Bevorzugt werden Zusammensetzungen mit hoher Oberfläche der Komponente a) von 40 bis 300 $m^2/g$, wobei gegebenenfalls noch Zinn- oder Wolframoxid enthalten sein können, und mit einer Komponente b), welche mit Mo, Cr, Sb und/oder Au dotiert ist.

Die katalytisch aktive Mischoxidmasse kann gegebenenfalls noch 10 bis 50 Gew.-%, bezogen auf das Gesamtgewicht der katalytisch aktiven Mischoxidmasse, inerte Verdünnungsmittel wie Siliciumdioxid, Siliciumcarbid oder Graphit enthalten.

Die katalytisch aktive Mischoxidmasse ist in einem Anteil von 1 bis 40 Gew.-%, vorzugsweise 5 bis 25 Gew.-%, jeweils bezogen auf das Gesamtgewicht aus Trägerkörper und aktiver Masse, als Schale auf die äußere Oberfläche des Trägerkörpers aufgebracht. Die Schichtdicke beträgt im allgemeinen 10 bis 2000 µm, vorzugsweise 100 bis 1000 µm. Der Schalenkatalysator kann auch mehrere, sich in der Zusammensetzung unterscheidende, Schichten enthalten. Es können auch ein oder mehrere Bestandteile der Aktivkomponente a) und b) in unterschiedlicher Konzentration in den einzelnen Schichten enthalten sein.

Im allgemeinen wird die katalytisch aktive Mischoxidmasse in einer Schicht aufgetragen. Zur Beeinflussung der Katalysatoraktivität und zur Verbesserung der Haftung am Trägerkörper können auch zwei oder mehr Schichten aufgetragen werden.

Bevorzugte Ausführungsformen mit mehreren Schichten sind solche in denen die innere Schicht nur Komponente a) enthält und die äußere Schicht die Komponenten a) und b). Bevorzugte Mehrschicht-Ausführungsformen sind auch solche, in denen die innere und äußere Schicht jeweils die Komponenten a) und b) enthalten, wobei vorzugsweise für die innere Schicht eine höhere spezifische Oberfläche für die Komponente a) gewählt wird, als für die äußere Schicht.

Geeignete Materialien für den inerten, unporösen Trägerkörper sind alle unter den Betriebsbedingungen der Gasphasenoxidation sich inert verhaltenden und über den Betriebszeitraum stabilen unporösen Materialien. Beispiele hierfür sind Steatit, Duranit, Siliciumcarbid, Magnesiumoxid, Siliciumoxid, Silikate, Aluminate, Metalle wie Edelstahl, sowie gegebenenfalls Mischungen aus diesen Stoffen. Bevorzugt wird keramisches Material wie Steatit.
Der Trägerkörper ist unporös, wobei unter unporös zu verstehen ist, daß die BET-Oberfläche des Trägerkörpers < 1 $m^2/g$ und die Porosität < 0,1 beträgt, mit
Porosität = [1 - (Dichte$_{Formkörper}$ / Dichte$_{Substanz}$)].

Die Formgestalt des inerten, unporösen Trägerkörpers ist beliebig. Beispiele für geeignete Formen sind Kugeln, Zylinder, Quader, Tori, Sättel, Spindeln, Wendeln. Die Grundkörper können auch eine oder mehrere Ausnehmungen wie Mulden, Rillen, Löcher, oder auch abstehende Teile wie Zapfen, Spitzen, Stege besitzen. Weitere Beispiele sind Ringe, Ringsegmente, Steg-Ringe, durchbohrte Kugeln, Kugelsegmente. Ebenfalls als Träger geeignet sind geordnete Packungen wie Monolithe oder Kreuzkanalstrukturen. Bevorzugt sind Trägerformen mit möglichst hoher geometrischer Oberfläche pro Volumen, beispielsweise Ringe.

Die Abmessungen der Trägerkörper sind durch die Reaktoren zur Gasphasenoxidation vorgegeben. Im allgemeinen haben die Formkörper eine Länge bzw. einen Durchmesser von 2 bis 20 mm.

Die Wanddicke, beispielsweise im Fall von Ringen oder Hohlzylindern, beträgt zweckmäßigerweise 0,1 bis 4 mm.

Zur Herstellung der Schalenkatalysatoren wird die katalytisch aktive Mischoxidmasse auf bekannte Art und Weise auf den Trägerkörper aufgebracht, beispielsweise durch Beschichten der Träger im Drehrohrofen mit einer wässerigen Aufschlämmung oder Dragieren. Vorteilhaft ist es, die Vormischung der aktiven Masse mit einem Binder zu versehen, der nach dem Aufbringen in der aktiven Schicht verbleibt um deren mechanische Stabilität zu verbessern. Besonders vorteilhaft ist es, die wässerige Suspension der Aktivkomponenten mit einer wässerigen Copolymerdispersion, vorzugsweise aus Vinylacetat/Vinyllaurat in einer Menge von 5 bis 40 Gew.-%, bezogen auf den Feststoffgehalt der Dispersion und die Summe der Trockengewichte von aktiver Masse und Dispersion, zu vermischen und diese Mischung in einem Sprühtrocknungsschritt auf die inerten, nicht porösen Trägerkörper aufzubringen.

Durch Wiederholen dieses Schrittes mit weiteren Sprühsuspensionen anderer Zusammensetzung können Katalysatoren mit schichtartigem Aufbau der aktiven Katalysatorschale hergestellt werden. Werden eine bzw. mehrere Komponenten der aktiven Masse während des Aufbringvorgangs in sich zeitlich ändernden Mengen zugeführt, werden katalytisch aktive Schichten erhalten, die eine stetige Änderung der Zusammensetzung entlang der Dickenachse zeigen.

Die nachfolgenden Beispiele sollen die Erfindung, ohne diese einzuschränken, näher erläutern.

Die Katalysatoren in den Beispielen wurden, in den in den Beispielen angegebenen Mengen, durch Vermahlen der Aktivkomponenten unter Zusatz von Wasser, anschließender Zugabe einer Copolymerdispersion aus Vinylacetat und Vinyllaurat mit 50 Gew.-% Feststoffanteil, und Versprühen der fertigen Suspension, unter Verdampfung des Wassers, auf 1000 g Steatit-Kugeln (4mm Durchmesser, Beispiel 1-27) bzw. 1200 g Steatitringe der Dimension 7*4*4 mm (Beispiel 28-30) (BET-Oberfläche < 0,01, Porosität < 0,01) aufgebracht.

Die Katalysatoren aus den Beispielen 3 bis 30 und den Vergleichsbeispielen 1 und 2 wurden zur Überprüfung ihrer Performance in einen Reaktor mit einem Reaktionsrohr mit einem Innendurchmesser von 12.5 mm gefüllt. Das Rohr wurde von außen mit einer zwangsumgewälzten Salzschmelze gekühlt. Soweit nichts anderes erwähnt wurden alle Umsetzungen bei $1 \times 10^5$ Pa Überdruck durchgeführt. Es wurden die in den Beispielen genannten Katalysatormengen in den Reaktor (Füllhöhe von 1400 mm) gefüllt. Falls nicht anderes in den Beispielen angegeben, wurde der Katalysator vor dem Betrieb für 6 h bei 410°C und einem Luftfluß von 220 Nl/h im Reaktionsrohr getempert.

Die Selektivität [in mol-%] wurde wie folgt berechnet:

$$\text{Essigsäureselektivität bezüglich gesamt } C_4\text{-Umsatz (mol-\%)}$$

$$= (((\text{mol/h Essigsäure in der Rohsäure})/2)/(\text{mol/h umgesetztes Buten} + \text{mol/h umgesetztes Butan}))*100$$

$$\text{Ameisensäureselektivität bezüglich gesamt } C_4\text{-Umsatz (mol-\%)}$$

$$= (((\text{mol/h Ameisensäure in der Rohsäure})/4)/(\text{mol/h umgesetztes Buten} + \text{mol/h umgesetztes Butan}))*100$$

Die Ausbeute wurde nach folgender Gleichung bestimmt:

$$\text{Essigsäureausbeute [Gew.-\%]} =$$

$$[(\text{kg abgeschiedene Essigsäure} / \text{kg eingesetztes Edukt}) \times 100]$$

Die Katalysatorzusammensetzungen sowie die Testbedingungen und Testergebnisse sind in Tabelle 1 zusammengefaßt.

Beispiel 1: (Oxidation von Butan zu Essigsäure)

Ein Kreislaufreaktor mit einem Reaktionsrohr von 6 m Länge und einem Innendurchmesser von 19 mm wurde mit einem Katalysator, bestehend aus 163 g $TiO_2$ (75 $M^2$/g BET-Obenfläche), 29 g $Sb_2O_3$, 16 g $V_2O_5$, 23 g Graphit auf Steatitringen der Dimension 7*4*4 mm, befüllt. Die Füllhöhe betrug 6000 mm. Das Rohr wurde von außen mit einem zwangsumgewälzten Ölbad gekühlt. Der Katalysator wurde vor dem Betrieb für 6 h bei 250°C und einem Luftfluß von 250 Nl/h im Reaktionsrohr getempert.
Die Reaktionsbedingungen wurden so gewählt, daß im Reaktor eine Temperatur von 205°C und ein Druck 10 bar(ü) herrschte. Die Zusammensetzung des Feeds betrug 120 g/h n-Butan, 136 g/h Sauerstoff und 700 g/h Wasserdampf.

Der Kreislauf wurde so eingestellt, daß ein Kreisgasfluß von 20 kg/h erreicht wurde. Der Butanumsatz betrug 53 %, der Sauerstoffumsatz 96 %. Die Essigsäureselektivität lag bei 65 mol-% und die Ameisensäureselektivität bei 5 mol-%.

Beispiel 2:

(Oxidation eines Gemisches aus n-Butan und 1-Buten zu Essigsäure)

Ein Kreislaufreaktor mit einem Reaktionsrohr von 6 m Länge und einem Innendurchmesser von 19 mm wurde mit einem Katalysator, bestehend aus 163 g $TiO_2$ (75 $M^2$/g BET-Oberfläche), 29 g $Sb_2O_3$, 16 g $V_2O_5$, 23 g Graphit auf Steatitringen der Dimension 7*4*4 mm, befüllt. Die Füllhöhe betrug 6000 mm. Das Rohr wurde von außen mit einem zwangsumgewälzten Ölbad gekühlt. Der Katalysator wurde vor dem Betrieb für 6 h bei 250°C und einem Luftfluß von 250 Nl/h im Reaktionsrohr getempert.

Die Reaktionsbedingungen wurden so gewählt, daß im Reaktor eine Temperatur von 200°C und ein Druck 10 bar(ü) herrschte. Die Zusammensetzung des Feeds betrug 120 g/h 1-Buten, 80 g/h n-Butan, 311 g/h Sauerstoff und 700 g/h Wasserdampf. Der Kreislauf wurde so eingestellt, daß ein Kreisgasfluß von 20 kg/h erreicht wurde. Der Butenumsatz betrug 99 %, der Butanumsatz 53 % und der Sauerstoffumsatz 96 %. Die Essigsäureselektivität lag bei 64 mol-% und die Ameisensäureselektivität bei 6 mol-%.

Beispiel 3: (Zweischalenkatalysator)

Zur Herstellung der 1. Sprühsuspension wurden 70,42 g Titandioxid (> 70 % Anatas-Modifikation) mit einer BET-Oberfläche von 48 $m^2$/g, 7,43 g Titandioxid (100 % Anatasmodifikation, BET 8 $m^2$/g), 14,67 g $V_2O_5$ und 900 ml vollentsalztem Wasser 20 h in einer Kugelmühle vermahlen. Zu der homogenen Suspension wurden danach 29 g einer Copolymerdispersion aus Vinylacetat und Vinyllaurat mit 50 Gew.-% Feststoffanteil zugegeben und innig verrührt. Zur Herstellung der 2. Sprühsuspension wurden 5 g Titandioxid (100 % Anatasmodifikation, BET 8 $m^2$/g), 3 g einer Copolymerdispersion aus Vinylacetat/Vinyllaurat mit 50 Gew.-% Feststoffanteil und 100 ml vollentsalztem Wasser innig verrührt. Auf 1000 g 4 mm Steatit-Kugeln wurde zuerst die 2. Sprühsuspension aufgebracht und getrocknet. Danach wurde die 1. Sprühsuspension aufgebracht und getrocknet.

202 g des Katalysators wurden in den Reaktor (Füllhöhe von 1400 mm) gefüllt. Bei einer Salzbadtemperatur von 213,5 °C wurde ein Umsatz von 88,6 % erreicht, die Essigsäureausbeute betrug 116,25 Gew.-%.

Beispiel 4: (Einschalenkatalysator, niedere Oberfläche)

211 g Titandioxid (> 70 % Anatas-Modifikation) mit einer BET-Oberfläche von 48 $m^2$/g wurden mit 44 g $V_2O_5$ und 1500 ml vollentsalztem Wasser 20 h in einer Kugelmühle vermahlen und nach Zugabe des Bindemittels auf die Steatit-Kugeln aufgebracht.

200 g Katalysator wurden in den Reaktor (Füllhöhe von 1400 mm) gefüllt. Bei einer Salzbadtemperatur von 198 °C wurde ein Umsatz von 88 % erreicht, die Essigsäureausbeute betrug 116,5 Gew.-%.

Beispiel 5: (hohe Schichtdicke)

297,1 g Titandioxid (> 70 % Anatas-Modifikation) mit einer BET-Oberfläche von 48 $m^2$/g wurden mit 51,3 g $V_2O_5$ und 1500 ml vollentsalztem Wasser 19 h in einer Kugelmühle vermahlen und nach Zugabe des Bindemittels auf die Steatit-Kugeln aufgebracht.

177 g Katalysator wurden in den Reaktor (Füllhöhe von 1400 mm) gefüllt. Das Reaktionsgas enthielt 24,8 Nl/h Wasserdampf. Bei einer Salzbadtemperatur von 196°C wurde ein Umsatz von 95 % erreicht, die Essigsäureausbeute betrug 113,5 Gew.-%.

Beispie1 6: (hohe Vanadiummenge)

135 g Titandioxid (Anatas-Modifikation) mit einer BET-Oberfläche von 75 $m^2$/g wurden mit 120 g $V_2O_5$ und 1400 ml vollentsalztem Wasser 24 h in einer Kugelmühle vermahlen und nach Zugabe des Bindemittels auf die Steatit-Kugeln aufgebracht.

168 g Katalysator wurden in den Reaktor (Füllhöhe von 1400 mm) gefüllt. Das Reaktionsgas enthielt 24.8 Nl/h Wasserdampf. Bei einer Salzbadtemperatur von 189°C wurde ein Umsatz von 92 % erreicht, die Essigsäureausbeute betrug 105 Gew.-%.

Beispiel 7: (Wolframdotierung)

186,2 g Titandioxid (Anatas-Modifikation mit 10 Gew.-% $WO_3$) mit einer BET-Oberfläche von 75 $m^2$/g wurden mit 68,9 g $V_2O_5$ und 700 ml vollentsalztem Wasser 120 h in einer Kugelmühle vermahlen und nach Zugabe des Bindemittels auf die Steatit-Kugeln aufgebracht.
192 g Katalysator wurden in den Reaktor (Füllhöhe von 1400 mm) gefüllt. Bei einer Salzbadtemperatur von 179°C wurde ein Umsatz von 95 % erreicht, die Hot-Spot-Temperatur betrug 190°C, die Essigsäureausbeute betrug 116,2 Gew.-%.

Beispiel 8: (Einschalenkatalysator, sehr hoher Oberfläche) 186,37 g Titandioxid (Anatas-Modifikation) mit einer BET-Oberfläche von 250 $m^2$/g wurden mit 68 g $V_2O_5$ und 1500 ml vollentsalztem Wasser 18 h in einer Kugelmühle vermahlen und nach Zugabe des Bindemittels auf die Steatit-Kugeln aufgebracht.
153,4 g Katalysator wurden in den Reaktor (Füllhöhe von 1400 mm) gefüllt.
Bei einer Salzbadtemperatur von 174°C wurde ein Umsatz von 96,2 % erreicht, die Essigsäureausbeute betrug 133 Gew.-%.

Beispiel 9: (Cäsium/Phosphor-Dotierung)

217,5 g Titandioxid (>70 % Anatas-Modifikation) mit einer BET-Oberfläche von 48 $m^2$/g wurden mit 37,6 g $V_2O_5$, 1.6 g Caesiumcarbonat, 4,8 g Ammoniumdihydrogenphosphat und 1000 ml vollentsalztem Wasser 48 h in einer Kugelmühle vermahlen und nach Zugabe des Bindemittels auf die Steatit-Kugeln aufgebracht.
166,8 g Katalysator wurden in den Reaktor (Füllhöhe von 1400 mm) gefüllt.
Bei einer Salzbadtemperatur von 221,5°C wurde ein Umsatz von 94 % erreicht, die Hot-Spot-Temperatur betrug 223°C, die Essigsäureausbeute betrug 105,5 Gew.-%.

Beispiel 10: (Molybdändotierung)

219,98 g Titandioxid (Anatas-Modifikation) mit einer BET-Oberfläche von 44,4 $m^2$/g wurden mit 31,33 g $V_2O_5$, 6.25 g Molybdäntrioxid und 1000 ml vollentsalztem Wasser 22 h in einer Kugelmühle vermahlen und nach Zugabe des Bindemittels auf die Steatit-Kugeln aufgebracht.
167 g Katalysator wurden in den Reaktor (Füllhöhe von 1400 mm) gefüllt.
Bei einer Salzbadtemperatur von 194°C wurde ein Umsatz von 93 % erreicht, die Essigsäureausbeute betrug 112,4 Gew.-%.

Beispiel 11: (Zweischalenkatalysator)

1. Sprühsuspension: 101,65 g Titandioxid (100 % Anatas-Modifikation) mit einer BET-Oberfläche von 75 $m^2$/g wurden mit 27,1 g $V_2O_5$ und 500 ml vollentsalztem Wasser 20 h in einer Kugelmühle vermahlen. Zu der homogenen Suspension wurden danach 43,50 g einer Copolymerdispersion aus Vinylacetat und Vinyllaurat mit 50 Gew.-% Feststoffanteil zugegeben und innig verrührt.
2. Sprühsuspension: 122,6 g Titandioxid (100 % Anatasmodifikation, BET 17 $m^2$/g), 7,50 g Vanadiumpentoxid und 500 ml vollentsalztes Wasser wurden 20 h in einer Kugelmühle vermahlen. Zu der homogenen. Suspension wurden danach 43,49 g einer Copolymerdispersion aus Vinylacetat und Vinyllaurat mit 50 Gew.-% Feststoffanteil zugegeben und innig verrührt. Auf 1000 g 4 mm Steatit-Kugeln wurde zuerst die 1.
Sprühsuspension aufgebracht. Danach wurde die 2.
Sprühsuspension aufgebracht.
167 g Katalysator wurden in den Reaktor (Füllhöhe von 1400 mm) gefüllt.
Bei einer Salzbadtemperatur von 185°C wurde ein Umsatz von 92 % erreicht, die Essigsäureausbeute betrug 116 Gew.-%.

Beispiel 12: (Mo/Na-Dotierung) .

100,0 g Titandioxid (100 % Anatas-Modifikation) mit einer BET-Oberfläche von 15 $m^2$/g wurden mit 5,32 g $V_2O_5$, 1,065 g Molybdäntrioxid, 0,245 g Natriumcarbonat und 1000 ml vollentsalztem Wasser 20 h in einer Kugelmühle vermahlen und nach Zugabe des Bindemittels auf die Steatit-Kugeln aufgebracht.
202 g Katalysator wurden in den Reaktor (Füllhöhe von 1400 mm) gefüllt.
Bei einer Salzbadtemperatur von 252°C wurde ein Umsatz von 92 % erreicht, die Essigsäureausbeute betrug 96,4 Gew.-%.

Beispiel 13: (geringer Vanadiumanteil)

225 g Titandioxid (100 % Anatas-Modifikation) mit einer BET-Oberfläche von 75 m$^2$/g wurden mit 30 g V$_2$O$_5$ und 1500 ml vollentsalztem Wasser 35 h in einer Kugelmühle vermahlen und nach Zugabe des Bindemittels auf 1000 g 4 mm Steatit-Kugeln aufgebracht.
158 g Katalysator wurden in den Reaktor (Füllhöhe von 1400 mm) gefüllt. Bei einer Salzbadtemperatur von 179°C wurde ein Umsatz von 95 % erreicht, die Essigsäureausbeute betrug 121,3 %.

Beispiel 14: (Schwefeldotierung)

188 g Titandioxid (Anatas-Modifikation) mit einer BET-Oberfläche von 140 m$^2$/g und einem Sulfatgehalt von 4,6 Gew.-% wurden mit 69 g V$_2$O$_5$ und 1500 ml vollentsalztem Wasser 100 h in einer Kugelmühle vermahlen und nach Zugabe des Bindemittels auf die Steatit-Kugeln aufgebracht.
161 g Katalysator wurden in den Reaktor (Füllhöhe von 1400 mm) gefüllt. Bei einer Salzbadtemperatur von 172,5°C wurde ein Umsatz von 95 % erreicht, die Essigsäureausbeute betrug 130 Gew.-%.

Beispiel 15: (hoher Mo-Gehalt)

225 g Titandioxid (Anatas-Modifikation) mit einer BET-Oberfläche von 75 m$^2$/g wurden mit 10 g V$_2$O$_5$, 20 g MoO$_3$ und 1500 ml vollentsalztem Wasser 68 h in einer Kugelmühle vermahlen und nach Zugabe des Bindemittels auf die Steatit-Kugeln aufgebracht.
162 g Katalysator wurden in den Reaktor (Füllhöhe von 1400 mm) gefüllt.
Bei einer Salzbadtemperatur von 183,5°C wurde ein Umsatz von 94,5 % erreicht, die Essigsäureausbeute betrug 120 Gew.-%.

Beispiel 16: (Antimondotierung)

225 g Titandioxid (Anatas-Modifikation) mit einer BET-Oberfläche von 75 m$^2$/g wurden mit 26,3 g V$_2$O$_5$, 10.5 g Sb$_2$O$_3$ und 2000 ml vollentsalztem Wasser 24 h in einer Kugelmühle vermahlen und nach Zugabe des Bindemittels auf die Steatit-Kugeln aufgebracht.
166 g Katalysator wurden in den Reaktor (Füllhöhe von 1400 mm) gefüllt.
Bei einer Salzbadtemperatur von 175°C wurde ein Umsatz von 94,4 % erreicht, die Essigsäureausbeute betrug 142 Gew.-%.

Beispiel 17: (Mo/Sb-Dotierung)

225 g eines Titandioxides mit einer BET-Oberfläche von 75 m$^2$/g wurden mit 20 g V$_2$O$_5$, 5 g MoO$_3$, 12 g Sb$_2$O$_3$ und 1500 ml vollentsalztem Wasser 14 h in einer Kugelmühle vermahlen und nach Zugabe des Bindemittels auf 1000 g Steatit-Kugeln aufgebracht.
166,2 g Katalysator wurden in den Reaktor (Füllhöhe von 1400 mm) gefüllt.
Bei einer Salzbadtemperatur von 178°C wurde ein Umsatz von 96 % erreicht, die Essigsäureausbeute betrug 125,2 Gew.-%.

Beispiel 18: (Sb-Dotierung)

225 g eines Titandioxides mit einer BET-Oberfläche von 75 m$^2$/g wurden mit 22 g V$_2$O$_5$, 40 g Sb$_2$O$_3$ und 1500 ml vollentsalztem Wasser 14 h in einer Kugelmühle vermahlen und nach Zugabe des Bindemittels auf 1000 g Steatit-Kugeln aufgebracht.
165,0 g Katalysator wurden in den Reaktor (Füllhöhe von 1400 mm) gefüllt.
Bei einer Salzbadtemperatur von 172°C wurde ein Umsatz von 94 % erreicht, die Essigsäureausbeute betrug 131,6 Gew.-%.

Beispiel 19: (Wismutdotierung)

225 g Titandioxid (100 % Anatas-Modifikation) mit einer BET-Oberfläche von 75 m$^2$/g wurden mit 26,3 g V$_2$O$_5$, 10,5 g Bi$_2$O$_3$ und 1500 ml vollentsalztem Wasser 48 h in einer Kugelmühle vermahlen und nach Zugabe des Bindemittels auf die Steatit-Kugeln aufgebracht.

151 g Katalysator wurden in den Reaktor (Füllhöhe von 1400 mm) gefüllt.

Bei einer Salzbadtemperatur von 179°C wurde ein Umsatz von 90,2 % erreicht, die Essigsäureausbeute betrug 120 Gew.-%.

Beispiel 20: (Tellurdotierung)

225 g Titandioxid (100 % Anatas-Modifikation) mit einer BET-Oberfläche von 75 m$^2$/g wurden mit 26,3 g $V_2O_5$, 10.5 g $TeO_2$ und 2000 ml vollentsalztem Wasser 47 h in einer Kugelmühle vermahlen und nach Zugabe des Bindemittels auf die Steatit-Kugeln aufgebracht.

159 g Katalysator wurden in den Reaktor (Füllhöhe von 1400 mm) gefüllt.

Bei einer Salzbadtemperatur von 203°C wurde ein Umsatz von 88 % erreicht, die Essigsäureausbeute betrug 103 Gew.-%.

Beispiel 21: (Mangandotierung)

225 g Titandioxid (100 % Anatas-Modifikation) mit einer BET-Oberfläche von 75 m$^2$/g wurden mit 26,3 g $V_2O_5$, 10,5 g $MnO_2$ und 1500 ml vollentsalztem Wasser 19 h in einer Kugelmühle vermahlen und nach Zugabe des Bindemittels auf die Steatit-Kugeln aufgebracht.

158 g Katalysator wurden in den Reaktor (Füllhöhe von 1400 mm) gefüllt.

Bei einer Salzbadtemperatur von 194°C wurde ein Umsatz von 90 % erreicht, die Essigsäureausbeute betrug 111,3 Gew.-%.

Beispiel 22: (Kupfer-Dotierung)

225 g Titandioxid (100 % Anatas-Modifikation) mit einer BET-Oberfläche von 75 m$^2$/g wurden mit 26,3 g $V_2O_5$, 1,42 g

$Cu(NO_3)_2X3H_2O$ und 1200 ml vollentsalztem Wasser 19 h in einer Kugelmühle vermahlen und nach Zugabe des Bindemittels auf die Steatit-Kugeln aufgebracht.

159 g Katalysator wurden in den Reaktor (Füllhöhe von 1400 mm) gefüllt.

Bei einer Salzbadtemperatur von 180°C wurde ein Umsatz von 94 % erreicht, die Essigsäureausbeute betrug 120 Gew.-%.

Beispiel 23: (Zinkdotierung)

225 g Titandioxid (100 % Anatas-Modifikation) mit einer BET-Oberfläche von 75 m$^2$/g wurden mit 26,3 g $V_2O_5$, 1,11 g Zink(II)acetat-dihydrat und 1500 ml vollentsalztem Wasser 43 h in einer Kugelmühle vermahlen und nach Zugabe des Bindemittels auf die Steatit-Kugeln aufgebracht.

157 g Katalysator wurden in den Reaktor (Füllhöhe von 1400 mm) gefüllt.

Bei einer Salzbadtemperatur von 178,5°C wurde ein Umsatz von 94,6 % erreicht, die Essigsäureausbeute betrug 124,2 Gew.-%.

Beispiel 24: (Golddotierung)

225 g Titandioxid (100 % Anatas-Modifikation) mit einer BET-Oberfläche von 75 m$^2$/g wurden mit 26,3 g $V_2O_5$, 0,94 g

Tetrachlorogoldsäure und 1500 ml vollentsalztem Wasser 46 h in einer Kugelmühle vermahlen und nach Zugabe des Bindemittels auf die Steatit-Kugeln aufgebracht.

162 g Katalysator wurden in den Reaktor (Füllhöhe von 1400 mm) gefüllt.

Bei einer Salzbadtemperatur von 182°C wurde ein Umsatz von 95,8 % erreicht, die Essigsäureausbeute betrug 128,4 Gew.-%.

Beispiel 25: (Chromdotierung)

225 g Titandioxid (100 % Anatas-Modifikation) mit einer BET-Oberfläche von 75 m$^2$/g wurden mit 24 g $V_2O_5$, 2,9 g

Chromtrioxid und 1500 ml vollentsalztem Wasser 22 h in einer Kugelmühle vermahlen und nach Zugabe des Bindemittels auf die Steatit-Kugeln aufgebracht.

174,8 g Katalysator wurden in den Reaktor (Füllhöhe von 1400 mm) gefüllt.

Bei einer Salzbadtemperatur von 184°C wurde ein Umsatz von 95 % erreicht, die Essigsäureausbeute betrug 128 Gew.-%.

Beispiel 26: (Ti/Sn-Katalysator)

200 g Titandioxid (100 % Anatas-Modifikation) mit einer BET-Oberfläche von 75 m$^2$/g wurden mit 70 g Zinntetrachlorid-Pentahydrat und 26,3 g $V_2O_5$ und 1500 ml vollentsalztem Wasser 46 h in einer Kugelmühle vermahlen und nach Zugabe des Bindemittels auf die Steatit-Kugeln aufgebracht.

160,1 g Katalysator wurden in den Reaktor (Füllhöhe von 1400 mm) gefüllt.

Bei einer Salzbadtemperatur von 191°C wurde ein Umsatz von 94 % erreicht, die Essigsäureausbeute betrug 130 Gew.-%:

Beispiel 27: (Ti/Zr-Katalysator)

171 g eines über ein Sol-Gel-Verfahren hergestellten Ti/Zr-Mischoxides (9 Gew.-% $ZrO_2$) mit einer BET-Oberfläche von 75 m$^2$/g wurden mit 15,2 g $V_2O_5$, 3,8 g $MoO_3$, 9,1 g $Sb_2O_3$ und 1000 ml vollentsalztem Wasser 14 h in einer Kugelmühle vermahlen und nach Zugabe des Bindemittels auf 760 g der Steatit-Kugeln aufgebracht.

162,4 g Katalysator wurden in den Reaktor (Füllhöhe von 1400 mm) gefüllt.

Bei einer Salzbadtemperatur von 201°C wurde ein Umsatz von 93 % erreicht, die Essigsäureausbeute betrug 112 Gew.-%.

Beispiel 28: (Ti/Zr-Katalysator)

171 g eines über ein Sol-Gel-Verfahren hergestellten Ti/Zr-Mischoxides (9 Gew.-% $ZrO_2$) mit einer BET-Oberfläche von 110 m$^2$/g wurden mit 36,8 g $V_2O_5$, 9,2 g $MoO_3$, 21,3 g $Sb_2O_3$ und 1000 ml vollentsalztem Wasser 14 h in einer Kugelmühle vermahlen und nach Zugabe des Bindemittels auf 760 g der Steatit-Kugeln aufgebracht.

155,5 g Katalysator wurden in den Reaktor (Füllhöhe von 1400 mm) gefüllt.

Bei einer Salzbadtemperatur von 199°C wurde ein Umsatz von 95 % erreicht, die Essigsäureausbeute betrug 115 Gew.-%.

Beispiel 29: (Niob-Dotierung)

151 g eines über ein Sol-Gel-Verfahren hergestellten Ti/Nb-Mischoxides (9 Gew.-% $Nb_2O_5$) mit einer BET-Oberfläche von 70 m$^2$/g wurden mit 14,5 g $V_2O_5$, 3,6 g $MoO_3$, 8,7 g $Sb_2O_3$ und 800 ml vollentsalztem Wasser 14 h in einer Kugelmühle vermahlen und nach Zugabe des Bindemittels auf 700 g der Steatit-Kugeln aufgebracht.

169,5 g Katalysator wurden in den Reaktor (Füllhöhe von 1400 mm) gefüllt.

Bei einer Salzbadtemperatur von 219°C wurde ein Umsatz von 96 % erreicht, die Essigsäureausbeute betrug 105 Gew.-%.

Beispiel 30: (Ca/Mo/Sb/SO$_4$-Dotierung)

225 g eines Titandioxides mit einer BET-Oberfläche von 75 m$^2$/g wurden mit 20 g $V_2O_5$, 5 g $MoO_3$, 12 g $Sb_2O_3$, 38 g $CaSO_4*2H_2O$ und 1500 ml vollentsalztem Wasser 14 h in einer Kugelmühle vermahlen und nach Zugabe des Bindemittels auf 1000 g Steatit-Kugeln aufgebracht.

158,5 g Katalysator wurden in den Reaktor (Füllhöhe von 1400 mm) gefüllt.

Bei einer Salzbadtemperatur von 181°C wurde ein Umsatz von 96 % erreicht, die Essigsäureausbeute betrug 120,3 Gew.-%.

Vergleichsbeispiel 1: (Vollkontakt-Katalysator)

200 g Titandioxid (100 % Anatas-Modifikation) mit einer BET-Oberfläche von 8 m$^2$/g wurden mit 8,1 g $V_2O_5$ und 10 g Graphit vermischt, vermahlen, gesiebt und zu zylindrischen Tabletten (4x4 mm) verpreßt.

155 g Katalysator wurden in den Reaktor (Füllhöhe von 1400 mm) gefüllt.

Bei einer Salzbadtemperatur von 247°C wurde ein Umsatz von 90 % erreicht, die Essigsäureausbeute betrug 77 Gew.-%.

Vergleichsbeispiel 2: (nach DE-A 2235103)

Ein poröser (Porosität = 0,65) alpha-Aluminiumoxidträger (irregulärer Bruch) wurde mit einer gemäß dem Beispielkatalysator 1 der DE-A 2235103 präparierten, salzsauren Vanadium/Titan-Lösung im Vakuum imprägniert und entsprechend diesem Beispiel getrocknet und calciniert.

134,5 g des auf 4 mm gesiebten Bruches wurden in den Reaktor (Füllhöhe 1400 mm) gefüllt. Bei einer Salzbadtemperatur von 200°C wurde ein Butenumsatz von 96 % erreicht, die Essigsäureausbeute betrug nur 94 Gew.-%. Als Nebenprodukte traten deutliche Mengen an Maleinsäure und Propionsäure auf.

TABELLE 1:

| Bsp. | Katalysator | Reaktions-bedingungen | Temperatur [°C] | Umsatz [%] | Ausbeute [%] |
|---|---|---|---|---|---|
| 3 | 2-Schalen | Standard | 213,5 | 88,6 | 116,25 |
| 4 | wenig Oberfläche | Standard | 198,0 | 88,0 | 116,5 |
| 5 | dicke Schicht | viel Dampf | 196,0 | 95,0 | 113,5 |
| 6 | hohe V-Menge | viel Dampf | 189,0 | 92,0 | 105,0 |
| 7 | W-Dotierung | Standard | 179,0 | 95,0 | 116,2 |
| 8 | hohe Oberfläche | Standard | 174,0 | 96,2 | 133,0 |
| 9 | Cs/P-Dotierung | Standard | 221,5 | 94,0 | 105,5 |
| 10 | Mo-Dotierung | Standard | 194,0 | 93,0 | 112,4 |
| 11 | 2-Schalen | Standard | 185,0 | 92,0 | 116,0 |
| 12 | Mo/Na-Dotierung | Standard | 252,0 | 92,0 | 96,4 |
| 13 | wenig Vanadium | Standard | 179,0 | 95,0 | 121,3 |
| 14 | S-Dotierung | Standard | 172,5 | 95,0 | 130,0 |
| 15 | hoher Mo-Gehalt | Standard | 183,5 | 94,5 | 120,0 |
| 16 | Sb-Dotierung | Standard | 175,0 | 94,4 | 142,0 |
| 17 | Mo/Sb-Dotierung | Standard | 178,0 | 96,0 | 125,2 |
| 18 | Sb-Dotierung | Standard | 172,0 | 94,0 | 131,6 |
| 19 | Bi-Dotierung | Standard | 179,0 | 90,2 | 120,0 |
| 20 | Te-Dotierung | Standard | 203,0 | 88,0 | 103,0 |
| 21 | Mn-Dotierung | Standard ' | 194,0 | 90,0 | 111,3 |
| 22 | Cu-Dotierung | Standard | 180,0 | 94,0 | 120,0 |
| 23 | Zn-Dotierung | Standard | 178,5 | 94,6 | 124,2 |
| 24 | Au-Dotierung | Standard | 182,0 | 95,8 | 128,4 |
| 25 | Cr-Dotierung | Standard | 184,0 | 95,0 | 128,0 |
| 26 | Ti/Sn-Katalysator | Standard | 191,0 | 94,0 | 130,0 |
| 27 | Ti/Zr-Katalysator | Standard | 201,0 | 93,0 | 112,0 |
| 28 | Ti/Zr-Katalysator | Standard | 199,0 | 95,0 | 115,0 |
| 29 | Nb-Dotierung | Standard | 219,0 | 96,0 | 105,0 |
| 30 | Ca/Mo/Sb/SO4-Dotierung | Standard | 181,0 | 96,0 | 120,3 |
| V1 | Vollkontakt | Standard | 247,0 | 90,0 | 77,0 |
| V2 | DE-A 2235103 | Standard | 200,0 | 96,0 | 94,0 |

**Patentansprüche**

**1.** Verfahren zur Herstellung von Essigsäure durch Gasphasenoxidation von gesättigten $C_4$-Kohlenwasserstoffen und deren Gemische mit ungesättigten $C_4$-Kohlenwasserstoffen in einem Rohrreaktor unter Verwendung eines Schalenkatalysators, bestehend aus einem inerten unporösen Trägerkörper und einer auf die äußere Oberfläche des Trägerkörpers aufgebrachten katalytisch aktiven Mischoxidmasse, welche

    a) ein oder mehrere Oxide aus der Gruppe Titandioxid, Zirkoniumdioxid, Zinndioxid, Aluminiumoxid und
    b) 0,1 bis 1,5 Gew.-%, bezogen auf das Gewicht der Komponente a) und pro $m^2/g$ spezifischer Oberfläche

der Komponente a), Vanadiumpentoxid enthält, wobei ein Gasgemisch, enthaltend Sauerstoff oder sauerstoffhaltiges Gas, ein oder mehrere $C_4$-Kohlenwasserstoffe und Wasserdampf, mit einem Volumenmischungsverhältnis $C_4$-Kohlenwasserstoff/Luft (Sauerstoff) von 0.2/99.8 bis 25/75 und einem Volumenmischungsverhältnis $C_4$-Kohlenwasserstoff/Wasserdampf von 1/1 bis 1/60, bei einer Temperatur von 100°C bis 400°C, und einem Überdruck von 0,2 bis 50 bar an dem Schalenkatalysator umgesetzt wird.

**2.** Verfahren nach Anspruch 1, dadurch gekennzeichnet, daß der Schalenkatalysator zonenweise in die Reaktionsrohre eingebracht wird.

**3.** Verfahren nach Anspruch 1 bis 2, dadurch gekennzeichnet, daß der Schalenkatalysator als Komponente a) zusätzlich noch ein oder mehrere Oxide aus der Gruppe Bor, Silicium, Hafnium, Niob, Wolfram, Lanthan und Cer, in einer Menge von 1 bis 30 Gew.-%, bezogen auf das Gesamtgewicht der Komponente a), enthält.

**4.** Verfahren nach Anspruch 1 oder 3, dadurch gekennzeichnet, daß im Schalenkatalysator als Komponente b) ein Teil des Vanadiumpentoxids durch ein oder mehrere Oxide von Molybdän, Chrom und Antimon ersetzt ist, und/oder als zusätzliche Komponente b) noch ein oder mehrere Oxide von Alkalimetallen, Erdalkalimetallen, Elementen der 5. und 6. Hauptgruppe des Periodensystems der Elemente (PSE) und der Übergangsmetalle enthalten sind.

**5.** Verfahren nach Anspruch 1 bis 4, dadurch gekennzeichnet, daß im Schalenkatalysator die katalytisch aktive Mischoxidmasse in einem Anteil von 1 bis 40 Gew.-%, bezogen auf das Gesamtgewicht aus Trägerkörper und aktiver Masse, als Schale, mit einer Schichtdicke von 10 bis 2000 μm, auf die äußere Oberfläche des Trägerkörpers aufgebracht ist.

**6.** Verfahren nach Anspruch 1 bis 5, dadurch gekennzeichnet, daß der Schalenkatalysator eine oder mehrere Schichten der katalytisch aktiven Mischoxidmasse enthält.

**7.** Verfahren nach Anspruch 1 bis 6, dadurch gekennzeichnet, daß der Schalenkatalysator mehrere Schichten enthält, wobei die innere Schicht nur Komponente a) enthält und die äußere Schicht die Komponenten a) und b).

**8.** Verfahren nach Anspruch 1 bis 5, dadurch gekennzeichnet, daß der Schalenkatalysator mehrere Schichten enthält, wobei die innere und äußere Schicht jeweils die Komponenten a) und b) enthalten, und für die innere Schicht eine höhere spezifische Oberfläche für die Komponente a) gewählt wird, als für die äußere Schicht.

**Claims**

**1.** Process for preparing acetic acid by gas-phase oxidation of saturated $C_4$-hydrocarbons and their mixtures with unsaturated $C_4$-hydrocarbons in a tube reactor using a coated catalyst comprising an inert nonporous support body and a catalytically active mixed oxide composition applied to the outer surface of the support body, that composition comprising

a) one or more oxides selected from the group consisting of titanium dioxide, zirconium dioxide, tin dioxide and aluminium oxide and

b) from 0.1 to 1.5% by weight, based on the weight of the component a) and per $m^2/g$ of specific surface area of the component a), of vanadium pentoxide, wherein a gas mixture comprising oxygen or oxygen-containing gas, one or -more $C_4$-hydrocarbons and water vapour and having a $C_4$-hydrocarbon/air (oxygen) volume ratio of from 0.2/99.8 to 25/75 and a $C_4$-hydrocarbon/water vapour volume ratio of from 1/1 to 1/60 is reacted over the coated catalyst at a temperature of from 100°C to 400°C and a gauge pressure of from 0.2 to 50 bar.

**2.** Process according to Claim 1, characterized in that the coated catalyst is introduced into the reaction tubes in zones.

**3.** Process according to Claim 1 or 2, characterized in that the coated catalyst further comprises, as component a), one or more oxides selected from the group consisting of the oxides of boron, silicon, hafnium, niobium, tungsten, lanthanum and cerium in an amount of from 1 to 30% by weight, based on the total weight of the component a).

**4.** Process according to Claim 1 or 3, characterized in that, in component b) of the coated catalyst, part of the vanadium pentoxide is replaced by one or more oxides of molybdenum, chromium and antimony, and/or one or more oxides of alkali metals, alkaline earth metals, elements of main groups V and VI of the Periodic Table of the Elements and the transition metals are present as additional component b).

5. Process according to any of Claims 1 to 4, characterized in that, in the coated catalyst, the catalytically active mixed oxide composition is applied in an amount of from 1 to 40% by weight, based on the total weight of support body and active composition, as a shell having a thickness of from 10 to 2000 µm to the outer surface of the support body.

6. Process according to any of Claims 1 to 5, characterized in that the coated catalyst comprises one or more layers of catalytically active mixed oxide composition.

7. Process according to any of Claims 1 to 6, characterized in that the coated catalyst comprises a plurality of layers and the inner layer comprises only component a) and the outer layer comprises components a) and b).

8. Process according to any of Claims 1 to 5, characterized in that the coated catalyst comprises a plurality of layers and the inner and outer layers each comprise the components a) and b) and the component a) in the inner layer has a higher specific surface area than the component a) in the outer layer.

**Revendications**

1. Procédé pour la préparation d'acide acétique par oxydation en phase gazeuse d'hydrocarbures en $C_4$ saturés et leurs mélanges avec des hydrocarbures en $C_4$ insaturés dans un réacteur tubulaire avec utilisation d'un catalyseur à coquille, constitué d'un corps support inerte non poreux et d'une masse d'oxydes mixtes catalytiquement active appliquée sur la surface extérieure du corps support, laquelle contient

   a) un ou plusieurs oxydes parmi le groupe constitué du dioxyde de titane, du dioxyde de zirconium, du dioxyde d'étain, de l'oxyde d'aluminium et
   b) 0,1 à 1,5% en poids, par rapport au poids du composant a) et par $m^2/g$ de surface spécifique du composant a), de pentoxyde de vanadium, un mélange gazeux, contenant de l'oxygène ou un gaz contenant de l'oxygène, un ou plusieurs hydrocarbures en $C_4$ et de la vapeur d'eau, présentant un rapport de mélange volumique hydrocarbure en $C_4$/ air (oxygène) de 0,2/99,8 à 25/75 et un rapport de mélange volumique hydrocarbure en $C_4$/ vapeur d'eau de 1/1 à 1/60, étant transformé à une température de 100°C à 400°C et à une surpression de 0,2 à 50 bars sur le catalyseur à coquille.

2. Procédé selon la revendication 1, caractérisé en ce que le catalyseur à coquille est introduit en zones dans les tubes de réaction.

3. Procédé selon la revendication 1 à 2, caractérisé en ce que le catalyseur à coquille contient en outre encore, comme composant a), un ou plusieurs oxydes parmi le groupe constitué par du bore, du silicium, de l'hafnium, du niobium, du tungstène, du lanthane et du cérium, en une quantité de 1 à 30% en poids par rapport au poids total du composant a).

4. Procédé selon la revendication 1 ou 3, caractérisé en ce que, dans le catalyseur à coquille, en tant que composant b), une partie du pentoxyde de vanadium est remplacée par un ou plusieurs oxydes de molybdène, de chrome et d'antimoine et/ou en tant que composant b) supplémentaire, encore un ou plusieurs oxydes de métaux alcalins, de métaux alcalino-terreux, d'éléments du 5ème et 6ème groupe principal du système périodique des éléments (SPE) et des métaux de transition sont contenus.

5. Procédé selon la revendication 1 à 4, caractérisé en ce que, dans le catalyseur à coquille, la masse d'oxydes mixtes catalytiquement active est appliquée, en une proportion de 1 à 40% en poids, par rapport au poids total de corps support et de masse active, en tant que coquille, avec une épaisseur de couche de 10 à 2000 µm, sur la surface extérieure du corps support.

6. Procédé selon la revendication 1 à 5, caractérisé en ce que le catalyseur à coquille contient une ou plusieurs couches de la masse d'oxydes mixtes catalytiquement active.

7. Procédé selon la revendication 1 à 6, caractérisé en ce que le catalyseur à coquille contient plusieurs couches, la couche interne ne contenant que le composant a) et la couche externe les composants a) et b).

8. Procédé selon la revendication 1 à 5, caractérisé en ce que le catalyseur à coquille contient plusieurs couches, la couche interne et la couche externe contenant à chaque fois les composants a) et b) et, pour le couche interne, une surface spécifique plus élevée que pour la couche externe étant choisie pour le composant a).